# EUROPÄISCHE PATENTSCHRIFT

(11) **EP 0 319 885 B1**
(45) Veröffentlichungstag und Bekanntmachung des Hinweises auf die Patenterteilung: **02.11.1994**
(21) Anmeldenummer: 88120262.6
(22) Anmeldetag: 05.12.1988
(51) Int. Cl.: A61N 5/10, A61B 6/06, G21K 1/04

(54) **Therapiesimulator**
Therapy simulator
Simulateur de thérapie

(30) Priorität: 11.12.1987 CH 4846/87
(43) Veröffentlichungstag der Anmeldung: 14.06.1989
(73) Patentinhaber: Varian International AG., CH-6300 Zug (CH)
(72) Erfinder: Schär, Hugo, CH-8416 Flaach (CH)
(74) Vertreter: Ottow, Jens M., Dr.

(56) Entgegenhaltungen:
- EP-A- 0 187 245
- FR-A- 2 440 014
- FR-A- 2 524 690
- US-A- 2 911 537

## Beschreibung

### Technisches Gebiet

Die Erfindung geht aus von einem Therapiesimulator gemäss dem Oberbegriff des Patentanspruches 1.

### Stand der Technik

Aus der Schrift FR-A-2 524 690 ist ein Therapiegerät bekannt, bei welchem eine Tiefenblende, die in mehrere Schichten aufgeteilt ist und die pro Schicht vier einzelne Blenden aufweist, Neutronenstrahlung abblendet. Bei dieser Tiefenblende werden alle Blenden und alle Schichten gleichzeitig parallel so bewegt, dass sich die in die Behandlungsebene projizierten Kanten der jeweiligen Blendenschicht mit gleicher Geschwindigkeit bewegen. Die verschiedenen Schichten wirken demnach wie eine einzige Blende. Die jeweils direkt übereinander liegenden Blenden sind über einen gemeinsamen Antrieb fest miteinander gekoppelt. Die Möglichkeiten den Blendenausschnitt zu variieren sind bei dieser Ausführung begrenzt, da ein Verfahren einzelner, bzw. eines gesamten Stapels übereinander liegender Blenden nicht vorgesehen ist, und auch ohne eine Beeinträchtigung der Abschirmwirkung nicht möglich ist. Eine separate Messblende ist hier nicht vorgesehen.

Aus dem Buch "Technik der medizinischen Radiologie" von Theodor Laubenberger (Deutscher Ärzte-Verlag GmbH, Köln, 4.Auflage, 1986) ist zudem ein Therapiesimulator bekannt für die Vorbereitung von Strahlentherapien. Bei diesem Therapiesimulator werden sämtliche Einstellungen an einem Bedienpult angezeigt und die entsprechenden Daten wie Lage, Grösse des Bestrahlungsfeldes und Winkelstellung werden protokolliert. Gleichzeitig werden Röntgenaufnahmen des Bestrahlungs- und des Umfeldes gemacht. Das Tiefenblendensystem und das Messblendensystem werden unabhängig voneinander ferngesteuert, so dass es von der Geschicklichkeit des Bedienenden abhängt, wie lange der zu untersuchende Patient der Röntgenstrahlung ausgesetzt ist und wie rasch gesunde Körperteile vor der Strahlung geschützt werden.

### DARSTELLUNG DER ERFINDUNG

Hier will die Erfindung Abhilfe schaffen. Die Erfindung, wie sie in den Ansprüchen gekennzeichnet ist, löst die Aufgabe, einen Therapiesimulator zu schaffen, bei welchem die Strahlenbelastung für den Patienten auf das unumgänglich Nötige beschränkt wird.

Die durch die Erfindung erreichten Vorteile sind im wesentlichen darin zu sehen, dass die Bedienung des Therapiesimulators durch die Kopplung der Bewegung der Messblende mit der der entsprechenden Tiefenblende wesentlich vereinfacht wird. Der Strahlerkopf kann sehr kompakt aufgebaut werden und da viele Gleichteile verwendet werden können, ist auch eine einfache Ersatzteillagerhaltung möglich. Ferner sind die bewegten Teile der Blendensysteme nur sehr geringem Verschleiss unterworfen, da ein Verkanten derselben in Führungsteilen ausgeschlossen ist.

Die weiteren Ausgestaltungen der Erfindung sind Gegenstände der abhängigen Ansprüche.

Die Erfindung, ihre Weiterbildung und die damit erzielten Vorteile werden nachstehend anhand der Zeichnungen, welche lediglich einen Ausführungsweg darstellen, näher erläutert.

### KURZE BESCHREIBUNG DER ZEICHNUNGEN

Es zeigen:
- Fig. 1: eine Prinzipskizze eines erfindungsgemässen Therapiesimulators,
- Fig. 2: eine Draufsicht auf die Blendensysteme und
- Fig. 3: eine Prinzipskizze eines Blendenpaares.

Bei allen Figuren sind gleich wirkende Elemente mit gleichen Bezugszeichen versehen.

### WEGE ZUR AUSFÜHRUNG DER ERFINDUNG

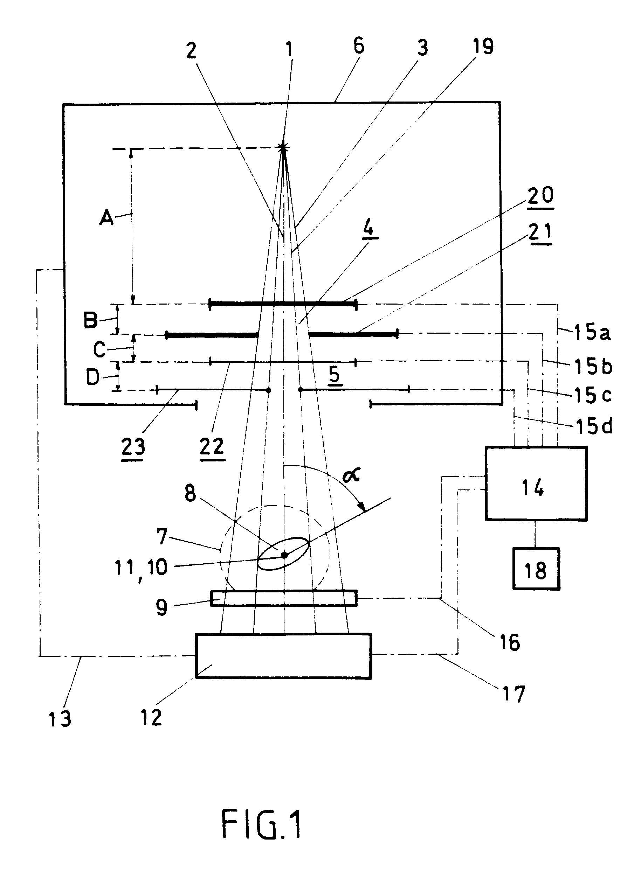

Fig. 1 zeigt einen erfindungsgemässen Therapiesimulator. Verkleidungsteile des Simulators sind nur angedeutet; die Grössenverhältnisse sind in dieser Prinzipskizze nicht masstäblich dargestellt. Als Strahlenquelle 1 kann eine Röntgenröhre vorgesehen werden, deren Strahlung in Richtung einer Hauptachse 2 einen Strahlungskegel 3 bildet. Die Strahlenquelle 1 ist zusammen mit einem Tiefenblendensystem 4 und einem Messblendensystem 5 in einem Strahlerkopf 6 angeordnet, welcher die Strahlenquelle 1 und die Blendensysteme trägt und gleichzeitig Streustrahlungen abschirmt. Der aus dem Strahlerkopf 6 austretende Strahlungskegel 3 durchdringt einen Patienten, dessen Querschnitt durch die gestrichelte Linie 7 angedeutet ist, mit einem Tumor 8. Der Patient ist auf einem verstellbaren Patientenlagerungstisch 9 gelagert. Häufig wird der Patient so gelagert, dass das Zentrum 10 des Tumors 8 auf der Hauptachse 2 und gleichzeitig auf der sich senkrecht zur Bildebene erstreckenden Schwenkachse 11 des Therapiesimulators liegt. Andere Lagerungsarten sind möglich entsprechend den jeweiligen Therapiezielen des behandelenden Arztes. Unter dem strahlendurchlässigen Patientenlagerungstisch 9 ist eine Bilderfassungseinheit 12 angeordnet. Der Strahlerkopf 6 und die Bilderfassungseinheit 12 sind gegeneinander entlang der Hauptachse 2 verschiebbar und werden durch das lediglich durch eine Wirkungslinie 13 angedeutete Gerüst des Therapiesimulators, welches als Schweiss- oder Gusskonstruktion ausgeführt sein kann, gehalten, zudem können sie gemeinsam motorgetrieben um die Schwenkachse 11 nach beiden Richtungen um einen Schwenkwinkel α geschwenkt werden. Der Schwenkbereich des Therapiesimulators ist dem eines zugehörigen Strahlentherapiegerätes angepasst. Dabei kann auch, falls erforderlich, der Patientenlagerungstisch 9 bewegt werden. Die Steuerung aller Bewegungen und deren Koordination erfolgt in einer zentralen Steuereinheit 14.

Durch die Wirkungslinien 15a, 15b, 15c und 15d ist angedeutet, dass die zentrale Steuerinheit 14 über Betätigungselemente auf die Blendensysteme 4, 5 einwirkt, und dass umgekehrt Stellungsmeldungen von den Blendensystemen 4, 5 in die Steuereinheit 14 zurückkommen. Die Wirkungslinie 16 deutet die Betätigungsmöglichkeit und die Stellungsmeldung für den Patientenlagerungstisch 9 an. Die entsprechende Verbindung zu einem für die Schwenkung um den Schwenkwinkel α zuständigen Antriebssystem ist nicht eingezeichnet. Die Wirkungslinie 17 zeigt die Möglichkeit an, dass koordiniert mit den übrigen Rückmeldungen auch Bildinformationen in die Steuereinheit 14 zurückfliessen und dort für eine gemeinsame Speicherung mit den Stellungsmeldungen in einem mit der Steuereinheit 14 verbundenen Speicher 18 aufbereitet werden können. Die Bildinformationen können auch auf Sichtgeräten sichtbar gemacht werden. Die Bewegungsdaten und Stellungsmeldungen werden dann entweder online oder mittels eines Datenträgers in einen Speicher des zugehörigen Strahlentherapiegerätes übertragen.

Ein Strahlungskegel 19, wie er durch die Position des Messblendensystems 5 in der dargestellten Ebene definiert ist, bestimmt das Bestrahlungsfeld im zugehörigen Strahlentherapiegerät. Wird nun der Therapiesimulator um den Schwenkwinkel α geschwenkt und dabei das Messblendensystem 5 so nachgeführt, dass der Tumor 8 stets gerade noch eingegrenzt ist, so wird im skizzierten Fall der Strahlenkegel 19 immer spitzer und das Bestrahlungsfeld in dieser Ebene damit kleiner.

Ein erstes Blendenpaar 20 des Tiefenblendensystems 4 ist im Abstand A von der Strahlenquelle 1 angeordnet und ein zweites Blendenpaar 21 im Abstand A + B. Das erste 20 und das zweite Blendenpaar 21 des Tiefenblendensystems 4 sind um 90° gegeneinander verdreht angeordnet. Im Abstand A + B + C von der Strahlenquelle 1 ist ein erstes Blendenpaar 22 des Messblendensystems 5 um 90° gegenüber dem Blendenpaar 21 verdreht angeordnet. Ein zweites Blendenpaar 23 des Messblendensystems 5 ist im Abstand A + B + C + D von der Strahlenquelle 1 angeordnet, es ist gegenüber dem Blendenpaar 22 um 90° verdreht. Die Abstände B, C und D sind hier jeweils gleich gewählt, da so eine kompakte Bauart des Strahlerkopfes 6 realisiert werden kann, sie können bei anderen Ausführungsformen auch verschieden gross sein. Ausserdem ist es möglich das erste Blendenpaar 22 des Messblendensystems 5 gleich auszurichten wie das Blendenpaar 21 des Tiefenblendensystems 4.

Das Blendenpaar 20 wird durch Betätigungselemente bewegt, die so ausgebildet sind, dass die Bewegungsgeschwindigkeit des Blendenpaares 20 proportional zum Abstand A von der Strahlenquelle 1 ist. Die Betätigungselemente für das Blendenpaar 21 bewegen dieses mit einer Geschwindigkeit proportional zum Abstand A + B, während das Blendenpaar 22 proportional zum Abstand A + B + C und das Blendenpaar 23 proportional zum Abstand A + B + C + D bewegt wird. Diese Geschwindigkeitsverteilung proportional zum Abstand des jeweiligen Blendenpaares von der Strahlenquelle 1 hat den Vorteil, dass sich die in die Bildebene der Bilderfassungseinheit 12 projizierten Blendensysteme 4, 5 dort mit gleicher Geschwindigkeit bewegen.

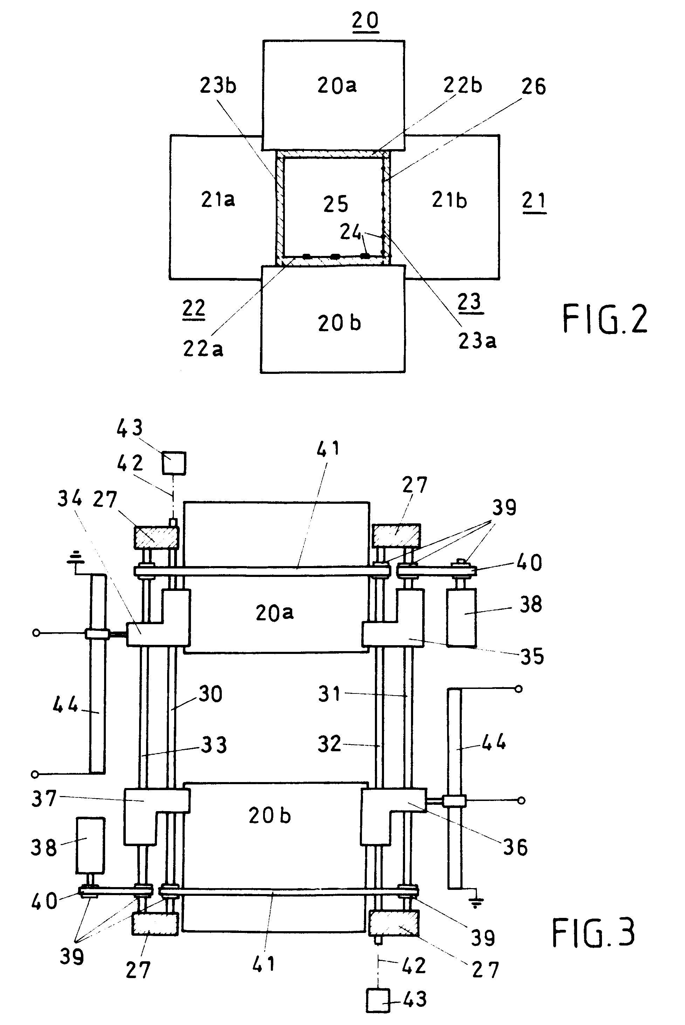

Jedes der Blendenpaare 20, 21, 22 und 23 besteht aus zwei einander gegenüberliegenden Blenden, wie aus Fig. 2 ersichtlich. Dabei sind dem Blendenpaar 20 die Blenden 20a und 20b zugeordnet, die aus Bleiplatten bestehen, ebenso dem Blendenpaar 21 die Blenden 21a und 21b, ebenfalls aus Blei. Dem Blendenpaar 22 sind die aus Wolfram- oder Molybdändraht bestehenden Blenden 22a und 22b zugeordnet und dem Blendenpaar 23 die entsprechenden Blenden 23a und 23b. Die Blenden 22a und 23a sind durch an ihnen befestigte, strahlungsundurchlässige Formstücke 24 unverwechselbar markiert. Durch die Blenden 22a, b und 23a, b wird ein Bestrahlungsfeld 25 eingerahmt. Zwischen den Blenden 22a, b 23a, b und den Blenden 20a, b und 21a, b wird eine schraffiert dargestellte Randzone 26 gebildet.

Jede der Blenden 20a, b, 21a, b, 22a, b und 23a, b wird einzeln angetrieben, beispielsweise durch einen Gleichstrommotor und falls es erforderlich sein sollte, kann auch jede Blende einzeln bewegt werden. In der Regel sind jedoch die Blenden 20a, b und 21a, b des Tiefenblendensystems 4 mit den zugeordneten Blenden 22b, a und 23b, a gekoppelt. Diese Koppelung wird durch die zentrale Steuereinheit 14 sichergestellt. Durch diese Koppelung wird die einmal eingestellte Breite der Randzone 26 in allen Blendenstellungen beibehalten und muss nicht jeweils von Hand nachgestellt werden. Eine gleichartige Koppelung könnte auch durch das Zwischenschalten von jeweils einem Diferentialgetriebe zwischen die einander zugeordneten Blenden erreicht werden.

In Fig. 3 ist das Blendenpaar 20 des Tiefenblendensystems 4 etwas detaillierter dargestellt. In fest mit dem Strahlenkopf 6 verbundenen Lagern 27 drehbar gelagerte, parallel in einer Ebene liegende Spindeln 30, 31, 32 und 33 tragen Halterungen 34, 35, 36 und 37, welche paarweise die Blenden 20a und 20b halten. Die Blende 20a wird dabei durch die Halterungen 34 und 35 gehalten und durch die Spindeln 30 und 31 bewegt. Es genügt hier lediglich die Spindel 31 mit einem Antrieb 38 über Ritzel 39 und einen Zahnriemen 40 anzutreiben, die zweite Spindel 30 wird über Ritzel 39 und einen zweiten Zahnriemen 41 mechanisch gekoppelt. Die Blende 20b wird durch die Halterungen 36 und 37 gehalten und durch die Spindeln 32 und 33, welche durch gleichartige Betätigungselemente angetrieben sind wie die Blende 20a, bewegt. In die Halterung 34 ist eine Gewindehülse eingepresst in welche die Spindel 30 eingreift, zudem ist eine zweite Bohrung vorgesehen, durch welche die Spindel 33 gleitet. Die anderen Halterungen 35, 36 und 37 sind entsprechend aufgebaut. Durch die Doppelfunktion der Spindeln 30, 31, 32, 33 als Betätigungselemente und gleichzeitig als Führungselemente erhält das Blendenpaar 20 eine grosse Stabilität und ein Verkanten der Halterungen 34, 35, 36, 37 ist ausgeschlossen, demzufolge wird der Verschleiss an den gleitenden und den miteinander in Eingriff stehenden Teilen des Blendenpaares 20 äusserst gering.

Die Steigung der Spindel 30, 31, 32 und 33 und die in deren Gegenstücken ist proportional zum Abstand A von der Strahlenquelle 1 gewählt worden. Die entsprechenden Teile des Blendenpaares 21 weisen eine Steigung proportional zum Abstand A + B auf. Die entsprechenden Teile der Blendenpaare 22 und 23 weisen Steigungen proportional zum Abstand A + B + C bzw. A + B + C + D auf. Die Spindeln 30 und 31 haben den gleichen Achsabstand voneinander wie die Spindeln 32 und 33.

Die jeweilige Position der Blendensysteme 4, 5 kann mittels Sensoren überwacht werden. Besonders genau kann die Position der Blenden durch zwei voneinander unabhängige Sensoren erfasst werden. Einer dieser Sensoren ist als mit einer der jeweils antreibenden Spindeln 30, 32, wie durch eine Wirkungslinie 42 angedeutet, gekoppelter Drehwinkelgeber 43 ausgebildet, ein anderer dieser Sensoren ist als ein den Gesamthub der jeweiligen Blende erfassendes Potentiometer 44 ausgebildet. In der zentralen Steuereinheit 14 werden die Positionsangaben beider Sensoren 43, 44 verglichen und, falls Uebereinstimmung besteht, im Speicher 18 gespeichert. Sollte ein Sensor gestört sein, so zeigt die Steuereinheit 14 dies an und der Schaden muss in Ordnung gebracht werden, denn falsch aufgezeichnete Daten könnten den betreffenden Patienten gefährten. In der Regel dürfte es jedoch genügen, lediglich die Position der Blenden des Messblendensystems 5 so genau zu überwachen, da ja mit diesen das genaue Bestrahlungsfeld abgegrenzt wird.

Die Wirkungsweise dieses Therapiesimulators soll kurz erläutert werden. Er dient zur Planung von Tumorbestrahlungen am therapiegerecht auf dem Patientenlagerungstisch 9 gelagerten Patienten. Die geometrischen Verhältnisse des Simulators werden an die des eigentlichen Strahlentherapiegerätes angeglichen, indem Strahlerkopf 6 und Bilderfassungseinheit 12 gegeneinander verschoben werden. Sollten diese, vom mechanischen Aufbau gegebenen Möglichkeiten nicht genügen, so können mit Hilfe der zentralen Steuereinheit 14 die im Speicher 18 gespeicherten Daten dem jeweiligen Strahlentherapiegerät angepasst werden. Der Patient wird beispielsweise auf dem Patientenlagerungstisch 9 so gelagert, dass das Zentrum 10 seines Tumors 8 auf der Hauptachse 2 der Strahlenquelle 1 und gleichzeitig auf der Schwenkachse 11 liegt, um welche Strahlerkopf 6 und Bilderfassungseinheit 12 gemeinsam schwenken. Während der Schwenkbewegung werden Röntgenaufnahmen des Tumors 8 gemacht und mittels des Messblendensystems 5 wird abhängig vom Schwenkwinkel α das Bestrahlungsfeld 25 definiert, welches später im eigentlichen Strahlentherapiegerät bestrahlt werden soll. Dabei ist es sehr vorteilhaft, wenn die Messblende mit der entsprechenden Tiefenblende gekoppelt ist, da beispielsweise beim Verkleinern des Bestrahlungsfeldes 25 mit der Bewegung der Messblende gleichzeitig die Tiefenblende nach innen fährt und sofort gesundes Gewebe vor der Röntgenstrahlung schützt. Die Bedienung des Therapiesimulators wird dadurch vereinfacht und der Schutz gesunden Körpergewebes verbessert. Es sind verschiedene Kopplungsmöglichkeiten der unterschiedlichen Blendensysteme denkbar, die je nach Grösse und Lage des Tumors im Körper des Patienten gewählt werden können. Ein Betrieb ohne diese Koppelung ist ebenfalls möglich, falls therapeutische Gründe dies erfordern sollten.

Beim Ausführungsbeispiel werden die Blenden durch Gleichstrommotoren mit vorgeschalteten Getrieben bewegt und die Antriebsenergie wird mittels Zahnriemen übertragen. Beim Erreichen von Endstellungen wird eine Rutschkupplung wirksam. Andere Antriebe, beispielsweise Linearmotoren oder drehzahlgeregelte Antriebe sind ebenfalls denkbar, ebenso andere Kraftübertragungen wie z.B. entsprechend den aufgezeigten Anforderungen an die Blendenbewegungen modifizierte Schnecken-Zahnstangentriebe.

Die durch die Blenden vorgegebenen Bildausschnitte müssen nicht immer rechteckig sein. Es ist durchaus denkbar auch Blenden einzusetzen, welche der Form des zu behandelnden Tumors besser angeglichen sind bzw. angeglichen werden können. Zudem ist es nicht erforderlich, den Tumor 8 im Bestrahlungsfeld 25 zu zentrieren, damit ist dem behandelnden Arzt eine vergleichsweise grosse Freiheit bei der Planung der Tumorbestrahlungen gegeben.

## Patentansprüche

1. Therapiesimulator mit einem eine vorzugsweise für die Abgabe von Röntgenstrahlung ausgelegte Strahlenquelle (1) enthaltenden Strahlerkopf (6), mit einer mit diesem verbundenen und gemeinsam schwenkbaren, eine Bildebene aufweisenden Bilderfassungseinheit (12), mit einem zwischen Strahlerkopf (6) und Bilderfassungseinheit (12) einfahrbaren, verstellbaren Patientenlagerungstisch (9), mit mindestens zwei im Strahlerkopf (6) in Richtung der Hauptachse (2) der Strahlenquelle (1) versetzt angeordneten Blendensystemen von denen mindestens ein erstes als Tiefenblendensystem (4) und mindestens ein zweites als Messblendensystem (5) zur Eingrenzung eines Bestrahlungsfeldes (25), ausgebildet ist, dadurch gekennzeichnet,
- dass mindestens eine Blende (20a) des Tiefenblendensystems (4) mit mindestens einer ihr auf der gleichen Seite des Bestrahlungsfeldes (25) zugeordneten Blende (22b) des Messblendensystems (5) gekoppelt ist, und
- dass jedes Paar von gekoppelten Blenden (20a,22b) gemeinsam und unabhängig von den übrigen Blenden des jeweiligen Blendensystems in die gleiche Richtung bewegbar ist, sodass sich die in die Bildebene der Bilderfassungseinheit (12) projizierten Blenden (20a,22b) in dieser Ebene mit gleicher Geschwindigkeit bewegen.

2. Therapiesimulator nach Anspruch 1, dadurch gekennzeichnet,
- dass jedes der mindestens zwei Blendensysteme aus vier paarweise einander gegenüberliegenden Blenden aufgebaut ist, und
- dass die Blendenpaare um jeweils 90° gegeneinander verdreht und jeweils in benachbarten Ebenen angeordnet sind.

3. Therapiesimulator nach Anspruch 2, dadurch gekennzeichnet,
- dass das unterste Blendenpaar (21) des Tiefenblendensystems (4) gegenüber dem in einer benachbarten Ebene darunter liegenden Blendenpaar (22) des Messblendensystems (5) ebenfalls um 90° verdreht angeordnet ist.

4. Therapiesimulator nach Anspruch 2, dadurch gekennzeichnet,
- dass die Blenden mittels in ihre Halterungen eingreifende Spindeln bewegbar ausgebildet sind, und
- dass die Steigung der jeweiligen Spindelgewinde proportional zum Abstand der jeweiligen Blenden von der Strahlenquelle (1) ist.

5. Therapiesimulator nach Anspruch 4, dadurch gekennzeichnet,
- dass für jede Blende (20a,20b) mindestens zwei antreibende Spindeln (30,31,32,33) vorgesehen sind,
- dass die mindestens vier antreibenden Spindeln (30,31,32,33) eines Blendenpaares (20) in einer Ebene parallel zueinander liegen, und
- dass die Halterungen (34,35) einer Blende (20a) sowohl durch die sie antreibenden mindestens zwei Spindeln (30,31) als auch durch die mindestens zwei Spindeln (32,33) der gegenüberliegenden Blende (20b) des Blendenpaares (20) geführt werden.

6. Therapiesimulator nach Anspruch 5, dadurch gekennzeichnet,
- dass die jeder Blende (20a,20b) zugeordneten mindestens zwei antreibenden Spindeln (30,31,32,33) den gleichen Abstand voneinander haben.

7. Therapiesimulator nach Anspruch 1, dadurch gekennzeichnet,
- dass mindestens die jeweilige Position aller Blenden (22a,22b,23a,23b) des Messblendensystems (5) in Abhängigkeit von der Schwenkbewegung des Strahlerkopfes (6) und der Bilderfassungseinheit (12) erfasst und reproduzierbar gespeichert wird.

8. Therapiesimulator nach Anspruch 7, dadurch gekennzeichnet,
- dass zwei voneinander unabhängige Sensoren die Position der jeweiligen Blende des Messblendensystems (5) erfassen, wobei ein erster Sensor als mit einer der mindestens zwei antreibenden Spindeln (30,31,32,33) gekoppelter Drehwinkelgeber (43) und ein zweiter Sensor als den gesamten Hub der Blende erfassendes Potentiometer (44) ausgebildet ist, und
- dass in einer Steuereinheit (10) die Positionsangaben beider Sensoren miteinander verglichen werden.

9. Therapiesimulator nach Anspruch 1 mit einem drahtförmig ausgebildeten Messblendensystem (5), dadurch gekennzeichnet,
- dass mindestens einer der Drähte des Messblendensystems (5) durch an ihm befestigte, strahlungsundurchlässige Formstücke (24) markiert ist.

## Claims

1. Therapy simulator having a radiator head (6) containing a radiation source (1), preferably designed for the discharge of X-radiation, having an image acquisition unit (12), which has an image plane and is connected to the said radiator head and able to swivel jointly with it, having an adjustable patient's couch (9), which can be moved in between radiator bead (6) and image acquisition unit (12), having at least two diaphragm systems, arranged in the radiator head (6) offset in the direction of the principal axis (2) of the radiation source (1), of which systems at least a first is designed as a depth diaphragm system (4) and at least a second is designed as a measuring diaphragm system (5), for the bounding of an irradiation field (25), characterized in that
- at least one diaphragm (20a) of the depth diaphragm system (4) is linked to at least one diaphragm (22b), assigned to it on the same side of the irradiation field (25), of the measuring diaphragm system (5), and
- each pair of linked diaphragms (20a, 22b) can be moved jointly and in the same direction, independently of the remaining diaphragms of the respective diaphragm system, so that the diaphragms (20a, 22b) projected into the image plane of the image acquisition unit (12) move in this plane at the same speed.

2. Therapy simulator according to Claim 1, characterized in that
- at least one of the at least two diaphragm systems is made up of four diaphragms opposite each other in pairs, and
- the diaphragm pairs are arranged turned through in each case 90° with respect to each other and in each case in neighbouring planes.

3. Therapy simulator according to Claim 2, characterized in that
- the lowermost diaphragm pair (21) of the depth diaphragm system (4) is arranged likewise turned through 90° with respect to the diaphragm pair (22) of the measuring system (5) lying in a neighbouring plane.

4. Therapy simulator according to Claim 2, characterized in that
- the diaphragms are designed so as to be moveable by means of spindles engaging in their holders, and
- the pitch of the respective spindle thread is proportional to the distance of the respective diaphragms from the radiation source (1).

5. Therapy simulator according to Claim 4, characterized in that
- at least two driving spindles (30, 31, 32, 33) are provided for each diaphragm (20a, 20b),
- the at least four driving spindles (30, 31, 32, 33) of a diaphragm pair (20) lie parallel to one another in a plane, and
- the holders (34, 35) of a diaphragm (20a) are guided by the at least two spindles (30, 31) driving them and by the at least two spindles (32, 33) of the opposite diaphragm (20b) of the diaphragm pair (20).

6. Therapy simulator according to Claim 5, characterized in that
- the at least two driving spindles (30, 31, 32, 33) assigned to each diaphragm (20a, 20b) have the same distance from one another.

7. Therapy simulator according to Claim 1, characterized in that
- at least the respective position of all diaphragms (22a, 22b, 23a, 23b) of the measuring diaphragm system (5) is detected and stored reproducibly in dependence on the swivelling movement of the radiator head (6) and of the image acquisition unit (12).

8. Therapy simulator according to Claim 7, characterized in that
- two mutually independent sensors detect the position of the respective diaphragm of the measuring diaphragm system (5), a first sensor being designed as an angle of rotation pickup (43), linked to the at least two driving spindles (30, 31, 32, 33), and a second sensor being designed as a potentiometer (44), detecting the overall travel of the diaphragm, and
- the position data of the two sensors are compared with one another in a control unit (10).

9. Therapy simulator according to Claim 1, having a measuring diaphragm system (5) designed in wire form, characterized in that
- at least one of the wires of the measuring diaphragm system (5) is marked by radiopaque shaped pieces (24) fastened to it.

## Revendications

1. Simulateur de thérapie avec une tête d'irradiation (6) contenant une source de rayonnement (1) conçue de préférence pour la production d'un rayonnement X, avec une unité de réception d'image (12) présentant un plan d'image, reliée à et basculant en commun avec cette tête d'irradiation (6), avec une table d'examen médical (9) réglable à introduire entre la tête d'irradiation (6) et l'unité de réception d'image (12), avec au moins deux systèmes d'écrans disposés dans la tête d'irradiation avec un décalage dans la direction de l'axe principal (2) de la source de rayonnement (1), dont au moins un premier est constitué par un système d'écrans de profondeur (4) et au moins un second est constitué par un système d'écrans de mesure (5) pour délimiter un champ d'irradiation (25), caractérisé en ce que
- au moins un écran (20a) du système d'écrans de profondeur (4) est couplé avec au moins un écran (22b) du système d'écrans de mesure (5) gui lui est associé du même côté du champ d'irradiation (25), et en ce que
- chaque paire d'écrans (20a, 22b) couplés est mobile en commun dans la même direction et indépendamment des autres écrans du système d'écrans considéré, de telle sorte que les écrans (20a, 22b) projetés dans le plan d'image de l'unité de réception d'image (12) se déplacent dans ce plan avec la même vitesse.

2. Simulateur de thérapie suivant la revendication 1, caractérisé en ce que
- chacun des au moins deux systèmes d'écrans est composé de quatre écrans disposés par paires l'un en face de l'autre, et en ce que
- les paires d'écrans sont chacune tournées de 90° l'une par rapport à l'autre et sont disposées dans des plans voisins.

3. Simulateur de thérapie suivant la revendication 2, caractérisé en ce que
- la paire d'écrans inférieure (21) du système d'écrans de profondeur (4) est également tournée de 90° par rapport à la paire d'écrans (22) du système d'écrans de mesure (5) située dans un plan voisin en dessous d'elle.

4. Simulateur de thérapie suivant la revendication 2, caractérisé en ce que
- les écrans peuvent être déplacés au moyen de broches engagées dans leurs supports, et en ce que
- le pas du filetage d'une broche est proportionnel à la distance entre l'écran correspondant et la source de rayonnement (1).

5. Simulateur de thérapie suivant la revendication 4, caractérisé en ce que
- pour chaque écran (20a, 20b) il est prévu au moins deux broches motrices (30, 31, 32, 33), en ce que
- les au moins quatre broches motrices (30, 31, 32, 33) d'une paire d'écrans (20) sont parallèles l'une à l'autre dans un plan, et en ce que
- les supports (34, 35) d'un écran (20a) sont guidés aussi bien par les au moins deux broches (30, 31) qui les entraînent qu'aussi par les au moins deux broches (32, 33) de l'écran opposé (20b) de la paire d'écrans (20).

6. Simulateur de thérapie suivant la revendication 5, caractérisé en ce que
- les au moins deux broches motrices (30, 31, 32, 33) associées à chaque écran (20a, 20b) présentent entr'elles la même distance.

7. Simulateur de thérapie suivant la revendication 1, caractérisé en ce que
- au moins la position respective de tous les écrans (22a, 22b, 23a, 23b) du système d'écrans de mesure (5) est déterminée en fonction du mouvement de basculement de la tête d'irradiation (6) et de l'unité de réception d'image (12) et est mémorisée de manière reproductible.

8. Simulateur de thérapie suivant la revendication 7, caractérisé en ce que
- deux détecteurs indépendants l'un de l'autre déterminent la position respective des écrans du système d'écrans de mesure (5), un premier détecteur étant constitué par un détecteur d'angle de rotation (43) couplé à une des au moins deux broches motrices (30, 31, 32, 33) et un second détecter étant constitué par un potentiomètre (44) déterminant la course totale de l'écran, et en ce que
- les données de position fournies par les deux détecteurs sont comparées les unes aux autres dans une unité centrale de commande (10).

9. Simulateur de thérapie suivant la revendication 1 avec un système d'écrans de mesure (5) en forme de fil, caractérisé en ce que
- au moins un des fils du système d'écrans de mesure (5) est marqué par des pièces de forme (24) imperméables au rayonnement qui lui sont fixées.
